# FASCICULE DE BREVET EUROPEEN

(11) **EP 0 499 505 B1**
(45) Date de publication et mention de la délivrance du brevet: **18.12.1996**
(21) Numéro de dépôt: 92400254.6
(22) Date de dépôt: 31.01.1992
(51) Int. Cl.: F16K 11/07, F16K 3/26

(54) **Valve de régulation pneumatique**
Pneumatisches Regelventil
Pneumatic control valve

(30) Priorité: 14.02.1991 FR 9101736
(43) Date de publication de la demande: 19.08.1992
(73) Titulaire: L'AIR LIQUIDE, SOCIETE ANONYME POUR L'ETUDE ET L'EXPLOITATION DES PROCEDES GEORGES CLAUDE, F-75321 Paris Cédex 07 (FR)
(72) Inventeur: Zapata, Richard, F-38360 Sassenage (FR); Arnault, Jean, F-38330 Saint Nazaire Les Eymes (FR)
(74) Mandataire: Le Moenner, Gabriel

(56) Documents cités:
- EP-A- 0 257 539
- EP-A- 0 301 825
- EP-A- 0 356 185
- DE-C- 46 877
- GB-A- 11 468
- US-A- 2 656 144
- US-A- 2 661 762

## Description

La présente invention a pour objet une valve de régulation de fluide comprenant un corps de valve définissant un logement intérieur de section sensiblement uniforme dans lequel est monté à coulissement un tiroir de valve comprenant une partie centrale coopérant avec le corps de valve pour établir une communication entre une première et une seconde zones du logement, et une partie d'extrémité coopérant sélectivement avec le logement pour séparer normalement la seconde zone d'une troisième zone dans le logement et pour établir une communication entre les seconde et troisième zones dans une position de course extrême du tiroir de valve vers la troisième zone, et des moyens d'actionnement du tiroir de valve.

Une valve de ce type est connue du document US-A-2.656.144. Des valves à tiroir, également à fonctionnement tout-ou-rien sont décrites dans les documents US-A-2.661.762 et EP-A-0.257.539.

La présente invention a pour objet de proposer une valve du type ci-dessus mais à commande de position sélective, de conception simple et robuste, susceptible d'une miniaturisation poussée, présentant une inertie réduite et autorisant une régulation à trois voies avec deux sorties de type proportionnel.

Pour ce faire, selon une caractéristique de l'invention, les moyens d'actionnement sont des moyens de commande de position sélective du tiroir dans le logement et la partie centrale du tiroir de valve présente une partie profilée coopérant avec au moins un premier joint porté par le corps de valve pour établir une communication modulable, selon la position du tiroir de valve, entre les première et seconde zones.

Selon une caractéristique plus particulière de l'invention, le logement comporte, à l'opposé de la première zone, une partie d'extrémité de section intérieure élargie dans laquelle la partie d'extrémité du tiroir de valve est reçue dans une position de course extrême du tiroir pour établir une communication modulable entre les deuxième et troisième zones.

Selon encore une autre caractéristique particulière de l'invention, les joints associés au tiroir de valve sont du type métalloplastique à expandeur.

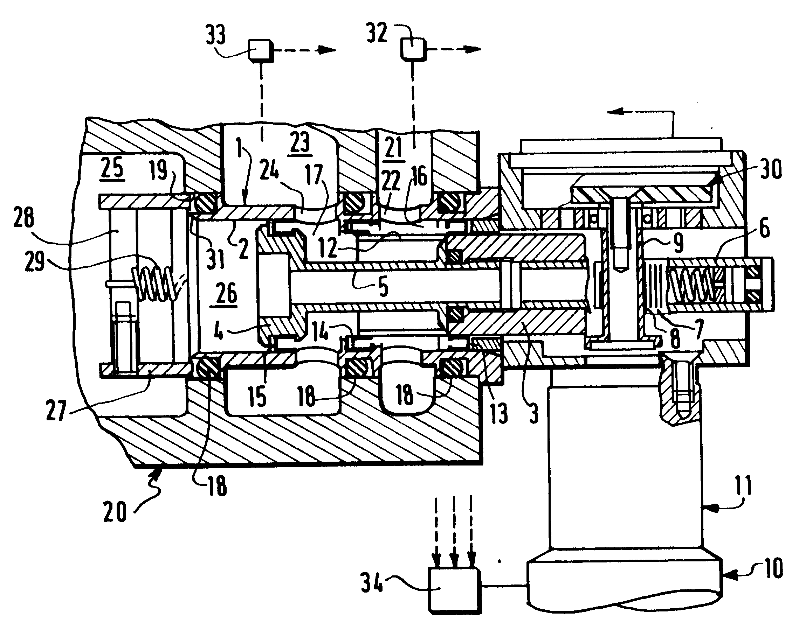

D'autres caractéristiques et avantages de la présente invention ressortiront de la description suivante d'un mode de réalisation donné à titre illustratif mais nullement limitatif, faite en relation avec les dessins annexés, sur lesquels la figure unique représente schématiquement un mode de réalisation d'une vanne de régulation pneumatique à trois voies selon l'invention.

La valve selon l'invention comprend un corps de valve 1, typiquement tubulaire, définissant un logement interne 2, typiquement un alésage, dans lequel est monté à coulissement un ensemble tiroir de valve comprenant une partie cylindrique 3 et une partie d'extrémité cylindrique 4 reliée à la partie centrale par une tige tubulaire 5. Dans l'exemple représenté, l'ensemble tiroir de valve comporte en outre une partie de tige arrière 6 de section quadratique dont une des faces est formée avec des dents de crémaillère 7 coopérant en engrènement avec un pignon 8 porté par un arbre 9 entraîné en rotation par un moteur électrique 10, du type à courant continu et accélération rapide, via un motoréducteur 11.

Selon un aspect de l'invention, la partie centrale 3 du tiroir de valve comporte une zone d'extrémité tubulaire dans la paroi de laquelle sont formées des lumières 12 présentant des profils déterminés en fonction des performances de régulation demandées. La portion non munie de lumières de la partie centrale 3 coopère en coulissement étanche avec un joint métalloplastique 13 monté dans une gorge de l'alésage 2 tandis que la portion d'extrémité munie des lumières 12 coopère avec un autre joint 14 également monté dans une gorge de l'alésage 2. La partie d'extrémité 4 du tiroir de valve porte un joint périphérique 15 coopérant avec la paroi de l'alésage 2. Les joints 13 et 14 délimitent, dans l'alésage 2, une première zone 16 alors que le joint 14 et le joint 15 délimitent dans l'alésage 2 une deuxième zone 17, les ouvertures 12 établissant une communication modulable, selon la position du tiroir de valve (3), entre ces deux zones 16 et 17.

Selon un aspect de l'invention, pour une meilleure tenue en durée et une inertie réduite de la valve, les joints 13, 14 et 15 sont des joints métalloplastiques semi-rigides à expandeur, tels que ceux commercialisés sous les appellations "Spring-ring" par la société Fluorocarbon ou "Enerseal" par la société Advanced Products.

Typiquement, le corps de valve 1 est monté, avec interposition de joints toriques 18, dans un alésage 19 d'un bloc de distribution 20 dans lequel sont formés un premier conduit ou conduit d'entrée 21 en communication permanente avec la première zone 16 par une série d'orifices 22 formés dans la paroi du corps de valve 2, un second conduit ou conduit de sortie 23 en communication permanente avec la deuxième zone 17 par une série d'orifices 24 formés dans la paroi du corps de valve 2, et un troisième conduit ou conduit d'échappement 25 dans lequel débouche une troisième zone 26 délimitée dans l'extrémité de l'alésage 2 par la partie d'extrémité 4 de l'ensemble tiroir de valve.

Selon un aspect de l'invention, le corps de valve 2 comporte, à son extrémité opposée au moteur 10, deux bras latéraux 27 s'étendant dans le troisième conduit 25 et pontés par une tige 28 à laquelle est accrochée une première extrémité d'un ressort hélicoidal de traction 29 s'étendant dans le tiroir de valve et dont l'autre extrémité est fixée intérieurement dans la partie de tige arrière 6 de façon à solliciter l'ensemble tiroir de valve dans la direction vers la tige 28 en rattrapant le jeu de la transmission entre le moteur 10 et l'ensemble tiroir de valve 3 et favorisant ainsi le contrôle des asservissements en positions du tiroir de valve assurés, par exemple, par un potentiomètre 30 couplé à l'arbre 9. Le ressort 29 permet en outre de placer le tiroir de valve 3 dans une position de sécurité en cas de défaillance des moyens de commande de ce tiroir de valve. Dans la troisième zone 26 de l'alésage 2, la paroi de ce dernier s'évase vers l'extérieur en formant un chanfrein 31 que le joint 15 de la partie d'extrémité 4 est susceptible de franchir dans une position de course extrême (vers la gauche sur le dessin) de l'ensemble tiroir de valve, en établissant ainsi une communication progressive entre la deuxième zone 17 et la troisième zone 26 alors que, dans cette position, les lumières 12 ont franchi le joint 14 qui isole ainsi la première zone 16 de la seconde zone 17.

La valve de régulation pneumatique selon l'invention trouve une application particulière dans un système de fourniture de gaz respiratoire à un passager d'aéronef, le premier conduit 21 étant relié, par un conduit d'alimentation, à une source de gaz contenant de l'oxygène, le deuxième conduit 23 étant relié, par un conduit d'utilisation, au masque du passager et le troisième conduit 25 communiquant avec l'atmosphère environnante. Dans un tel système, des capteurs de pression 32 et 33 détectent les pressions dans la conduite d'amenée 21 et dans la conduite d'utilisation 23, les signaux provenant des capteurs 32 et 33 ainsi que du potentiomètre 30 étant adressés à un bloc électronique 34 de commande du moteur électrique 10.

## Revendications

1. Valve de régulation de fluide, comprenant un corps de valve (1) définissant un logement intérieur de section sensiblement uniforme (2) dans lequel est monté à coulissement un tiroir de valve (3, 4) comprenant une partie centrale (3) coopérant avec le corps de valve pour établir une communication entre une première (16) et une seconde (17) zones du logement, et une partie d'extrémité (4) coopérant sélectivement avec le logement (2) pour séparer normalement la seconde zone (17) d'une troisième zone (26) dans le logement (2) et pour établir une communication entre les seconde (17) et troisième (26) zones dans une position de course extrême du tiroir de valve vers la troisième zone, et des moyens (10) d'actionnement du tiroir de valve, caractérisée en ce que les moyens d'actionnement sont des moyens de commande de position sélective du tiroir dans le logement et en ce que la partie centrale (3) du tiroir de valve présente une partie profilée (12) coopérant avec au moins un premier joint (14) porté par le corps de valve pour établir une communication modulable selon la position du tiroir de valve entre les première (16) et seconde (17) zones.

2. Valve selon la revendication 1, caractérisée en ce que la partie d'extrémité porte un deuxième joint (15) coopérant avec le logement (2).

3. Valve selon la revendication 1 ou 2, caractérisée en ce que le logement (2) comporte, à l'opposé de sa première zone (16), une partie de section intérieure élargie (31) dans laquelle est reçue la partie d'extrémité (4) du tiroir de valve dans la position de course extrême du tiroir de valve pour établir une communication modulable entre les deuxième (17) et troisième (26) zones.

4. Valve selon la revendication 3, caractérisée en ce que, dans la position de course extrême du tiroir de valve, la communication entre les première (16) et deuxième (17) zones est interrompue.

5. Valve selon l'une des revendications précédentes, caractérisée en ce que le corps de valve (1) est monté dans un bloc de distribution (20) comportant des premier (21), deuxième (23) et troisième (25) conduits communiquant respectivement avec les première (16), deuxième (17) et troisième (26) zones du logement (2) par des ouvertures (22, 24, 2) formées dans le corps de valve et en ce que le premier conduit (21) est relié à un circuit d'alimentation, le deuxième conduit (23) étant relié à un circuit d'utilisation et le troisième conduit (25) communiquant avec l'atmosphère environnante.

6. Valve selon l'une des revendications 2 à 5, caractérisée en ce que les premier (14) et second (15) joints sont des joints métalloplastiques à expandeur.

7. Valve selon l'une des revendications précédentes, caractérisée en ce que les moyens de commande comprennent un moteur électrique (10).

8. Valve selon la revendication 7, caractérisée en ce que le tiroir de valve comporte une crémaillère (7) coopérant avec un pignon (8) entraîné par le moteur (10).

9. Valve selon l'une des revendications 1 à 8, caractérisée en ce qu'elle comporte des moyens (30) de contrôle de position du tiroir de valve (3, 4) dans le logement (2).

10. Valve selon l'une des revendications 1 à 9, caractérisée en ce que le tiroir de valve (3, 4) est sollicité par un ressort de traction (29) vers la position de course extrême du tiroir de valve (3, 4).

## Patentansprüche

1. Fluidregelventil, umfassend einen Ventilkörper (1), der einen inneren Sitz mit im wesentlichen gleichförmigem Querschnitt (2) definiert, in welchem verschieblich ein Ventilschieber (3, 4) angeordnet ist, der einen Mittenabschnitt (3) umfaßt, welcher so mit dem Ventilkörper zusammenwirkt, daß eine Verbindung zwischen einem ersten (16) und einem zweiten (17) Bereich des Sitzes hergestellt wird, einen Endabschnitt (4), der selektiv mit dem Sitz (2) so zusammenwirkt, daß normalerweise der zweite Bereich (17) von einem dritten Bereich (26) in dem Sitz (2) getrennt wird und daß in einer Extremstellung des Ventilschiebers in Richtung des dritten Bereichs eine Verbindung zwischen dem zweiten (17) und dem dritten (26) Bereich hergestellt wird, und Mittel (10) zur Betätigung des Ventilschiebers, dadurch gekennzeichnet, daß die Betätigungsmittel Mittel zur selektiven Positionssteuerung des Schiebers in dem Sitz sind, und daß der Mittenabschnitt (3) des Ventilschiebers einen profilierten Abschnitt (12) aufweist, der so mit zumindest einer ersten Verbindungsstelle (14) an dem Ventilkörper zusammenwirkt, daß eine entsprechend der Position des Ventilschiebers zwischen dem ersten (16) und dem zweiten (17) Bereich beeinflußbare Verbindung hergestellt wird.

2. Ventil nach Anspruch 1, dadurch gekennzeichnet, daß der Endabschnitt eine zweite Verbindungsstelle (15) aufweist, die mit dem Sitz (2) zusammenwirkt.

3. Ventil nach Anspruch 1 oder 2, dadurch gekennzeichnet, daß der Sitz (2) seinem ersten Bereich (16) gegenüberliegend einen Abschnitt (31) mit erweitertem Innenquerschnitt umfaßt, in welchem der Endabschnitt (4) des Ventilschiebers in der Extremstellung des Ventilschiebers so Aufnahme findet, daß eine beeinflußbare Verbindung zwischen dem zweiten (17) und dem dritten Abschnitt (26) hergestellt wird.

4. Ventil nach Anspruch 3, dadurch gekennzeichnet, daß in der Extremstellung des Ventilschiebers die Verbindung zwischen dem ersten (16) und dem zweiten (17) Bereich unterbrochen ist.

5. Ventil nach einem der vorangehenden Ansprüche, dadurch gekennzeichnet, daß der Ventilkörper (1) in einem Verteilerblock (20) angeordnet ist, der eine erste (21), eine zweite (23) und eine dritte (25) Leitung umfaßt, die über in dem Ventilkörper ausgebildete Öffnungen (22, 24, 2) jeweils mit dem ersten (16), dem zweiten (17) und dem dritten (26) Bereich des Sitzes kommunizieren, und daß die erste Leitung (21) mit einem Speisekreis verbunden ist, die zweite Leitung (23) mit einem Nutzkreis verbunden ist und die dritte Leitung (25) mit der umgebenden Atmosphäre in Verbindung steht.

6. Ventil nach einem der Ansprüche 2 bis 5, dadurch gekennzeichnet, daß die erste (14) und die zweite (15) Verbindungsstelle metalloplastische Expander-Verbindungsstellen sind.

7. Ventil nach einem der vorangehenden Ansprüche, dadurch gekennzeichnet, daß die Steuermittel einen Elektromotor (10) umfassen.

8. Ventil nach Anspruch 7, dadurch gekennzeichnet, daß der Ventilschieber eine Zahnstange (7) umfaßt, die mit einem durch den Motor (10) angetriebenen Ritzel (8) zusammenwirkt.

9. Ventil nach einem der Ansprüche 1 bis 8, gekennzeichnet durch Mittel (30) zur Kontrolle der Position des Ventilschiebers (3, 4) in dem Sitz (2).

10. Ventil nach einem der Ansprüche 1 bis 9, dadurch gekennzeichnet, daß der Ventilschieber (3, 4) durch eine Zugfeder (29) in Richtung der Extremstellung des Ventilschiebers (3, 4) beansprucht wird.

## Claims

1. Fluid regulating valve, comprising a valve body (1) defining an internal housing with a substantially uniform cross section (2) in which a valve slide (3,4) is mounted so as to slide, comprising a central part (3) cooperating with the body of the valve to establish communication between a first (16) and a second (17) zone of the housing, and an end part (4) cooperating selectively with the housing (2) for separating normally the second zone (17) from a third zone (26) in the housing (2) and to establish communication between the second zone (17) and the third zone (26) in the end position of traverse of the valve slide towards the third zone, and means (10) for operating the valve slide, characterized in that the operating means are means for selectively controlling the position of the slide in the housing and in that the central part (3) of the valve slide has a shaped part (12) cooperating with at least a first seal (14) carried by the body of the valve to establish an adjustable communication according to the position of the valve slide between the first (16) and the second (17) zones.

2. Valve according to claim 1, characterized in that the end part carries a second seal (15) cooperating with the housing (2).

3. Valve according to claim 1 or 2, characterized in that the housing (2) comprises, on the opposite side to its first zone (16), a part with an enlarged internal cross section (31) in which the end part (4) of the valve slide is received in the end position of traverse of the valve slide to establish an adjustable communication between the second (17) and third (26) zones.

4. Valve according to claim 3, characterized in that, in the end position of traverse of the valve slide, communication between the first (16) and the second (17) zones is cut off.

5. Valve according to one of the preceding claims, characterized in that the body of the valve (1) is mounted in a distribution block (20) comprising first (21), second (23) and third (25) channels communicating respectively with the first (16), second (17) and third (26) zones of the housing (2) by openings (22, 24, 2) formed in the body of the valve and in that the first channel (21) is connected to a supply circuit, the second channel (23) being connected to a working circuit and the third channel (25) communicating with the surrounding atmosphere.

6. Valve according to one of claims 2 to 5, characterized in that the first (14) and second (15) seals are expanding metallo-plastic seals.

7. Valve according to one of the preceding claims, characterized in that the means of control comprise an electric motor (10).

8. Valve according to claim 7, characterized in that the valve slide comprises a rack (7) cooperating with a pinion (8) driven by the motor (10).

9. Valve according to one of claims 1 to 8, characterized in that it comprises means (30) for controlling the position of the valve slide (3, 4) in the housing (2).

10. Valve according to one of claims 1 to 9, characterized in that the valve slide (3, 4) is tensioned by an extension spring (29) towards the end position of traverse of the valve slide (3,4).
